# EUROPEAN PATENT APPLICATION

(11) **EP 1 762 248 A1**
(43) Date of publication of application: **14.03.2007**
(21) Application number: 05425630.0
(22) Date of filing: 09.09.2005
(51) Int. Cl.: A61K 45/06, A61P 37/00, A61P 41/00, A61K 31/409, A61K 38/13

(54) **Use of cobalt porphyrins in joint therapy with an immunosuppressant drug for treatment of transplant patients or autoimmune disorders**

(71) Applicant: CORIT Consorzio per la Ricerca sul, 35131 Padova (IT)
(72) Inventor: Cozzi, Emanuele, Via Passagio Gaudenzio 35131 PADOVA (IT); Seveso, Michela, Via Passagio Gaudenzio 35131 PADOVA (IT); Besenzon, Federica, Via Passagio Gaudenzio 35131 PADOVA (IT); Bommer, Jerry C., Via Passagio Gaudenzio 35131 PADOVA (IT)
(74) Representative: Long, Giorgio

(57) **Abstract**

The present invention relates to the use of metalloporphyrins for the preparation of a remedy for the treatment of patients having undergone organ or cell transplantation or patients affected by an autoimmune disorder, as well as a pharmaceutical composition containing said metalloporphyrins in joint therapy with an immunosuppressant.

## Description

The present invention relates to the use of metalloporphyrins for the preparation of a remedy for the treatment of patients having undergone organ or cell transplantation or patients affected by an autoimmune disorder, as well as a pharmaceutical composition containing said metalloporphyrins.

Immunomodulant or immunosuppressant drugs constitute a family of compounds capable of interfering with the operation of the immune system, predominantly exercising an inhibitory action on the patient's immune system. Such drugs are widely used in the treatment of both patients subjected to organ transplantation and patients affected by autoimmune disorders in which the immune system is no longer capable of discriminating between the individuals own biological material and foreign material, and is hence constantly active.

While the global number of transplant patents corresponds to around 40,000 per year, it is important to remember that autoimmune disorders affect up to 5% of the population.

There are numerous classes of immunomodulant drugs capable of intervening in the various stages of the immune response, such as antigen recognition (steroids), the interaction between antigen presenting cells and lymphocytes and the effector signals (cyclosporin A, FK506, anti-CD3, anti-IL2R, Jak3 inhibitors), cell proliferation (alkylating agents, antifolates, AZA, MMF) and cell migration (FTY720).

For some of the above drugs, the mechanisms of action have been widely studied, while for others, the specific molecular targets have still to be elucidated. Furthermore, although some compounds act selectively, for example by blocking activated T cells (anti-IL2R, Jak3 inhibitors), other drugs interact with generic cellular mechanisms (alkylating agents, MMF) and may hence interfere with the integrity of various tissues and organs, giving rise to serious side effects. Furthermore, even though there are specific therapeutic concentrations recommended for each drug to which the desired therapeutic effect is associated, essentially in the absence of, or with few side effects, there is always significant variability in the response between patients, thereby exacerbating the problem.

Combinations of immunosuppressant drugs are frequently used in immunosuppressant therapies, which, by interfering with different aspects of the immune response, may result in a potentiated pharmacological effect. However, this type of therapy may give rise to undesired side effects associated with individual drugs or to combinations of the various drugs used. For example, the same pro-apoptotic effect which an immunomodulator elicits on lymphocytes may also be observed on the cells of endothelial tissues, which hence become damaged. The consequence, in transplant or autoimmune patients, is the onset of disorders such as glomerulosclerosis, diabetes, hypertension and serious vasculopathies.

Hence, it would be desirable to have access to drugs with protective effects (or which permit reduced dosages) in relation to the side effects caused by immunomodulating drugs.

Porphyrins are a family of molecules capable of inducing the expression of the enzyme heme oxygenase-1 (HO-1). HO-1 is the enzyme responsible for the degradation of heme, and is considered to be a potent cytoprotective and anti-apoptotic enzyme which can suppress the pro-inflammatory responses associated with the immunosuppressant drug-mediated activation of endothelial cells. It has been shown that transplanted organs can express high levels of protective genes capable of attenuating the inflammatory reactions which follow the activation of endothelial cells, according to a mechanism known as accommodation.

In a xenotransplantation model where a mouse heart has been transplanted into a rat, immunosuppressed with cyclosporin A (CsA) and CVF (Cobra Venom Factor), the survival of the transplant was indefinite. By inhibiting the expression of HO-1 through the use of inhibitor molecules, or by using knockout mice for the gene encoding HO-1, the organ was rapidly rejected. Even rat kidney allograft models show higher survival and reduced transplant damage in rats treated with porphyrins such as cobalt (III) protoporphyrin (CoPP).

Also, in cases of experimental autoimmune encephalomyelitis (an experimental model of human multiple sclerosis) induction of HO-1 expression seems important in the attenuation of oxidative phenomena, with the inhibition of the disease.

Besides the protective role, it is known that HO-1 expression also intervenes in modulating T cell activation. HO-1 knockout mice have a greater number of circulating lymphocytes. More recently, it has been shown that induction of HO-1 expression is associated with the inhibition of human CD4 T lymphocyte proliferation. Furthermore, induction of HO-1 expression promotes (AICD) activation induced cell death (apoptosis) of CD4 T lymphocytes.

It has now been surprisingly discovered that the use of metalloporphyrins in association with immunosuppressant drugs not only promotes a reparative and possibly protective effect in relation to endothelial cells, frequently the target of the tissue damage induced by rejection and immunosuppression, but also has a synergistic effect on the mechanisms of apoptosis towards the cells of the immune system.

In other words, the apoptotic effect induced by the combined administration of the immunosuppressor and the metalloporphyrin is greater than the simple sum of the effects induced by the two drugs when administered separately.

This synergistic action allows the establishment of a therapy where the immunosuppressant drug is administered at a lower dose than the dose predetermined to be therapeutically effective, or determined throughout the course of therapy on the basis of the immunosuppressant drug plasma levels, for the treatment of transplant patients or patients affected by autoimmune disorders when the immunosuppressant drug is administered alone.

Preferably, said immunosuppressant drug will be administered at a dose at least 25% lower, with respect to the therapeutically effective predetermined dose, or determined throughout the course of the treatment on the basis of plasma levels of the immunosuppressant drug, for the treatment of transplant patients or patients affected by autoimmune disorders when the immunosuppressant drug is administered alone. More preferably, said immunosuppressant drug will be administered at a dose at least 30% lower with respect to said therapeutically effective dose of the drug.

In the present patent application, the term "immunosuppressant drug", is intended to mean both a single active ingredient and a mixture of several active ingredients having an immunomodulatory effect.

Active ingredients which may be used alone or in combination with an immunosuppressant drug according to the invention may be selected, non-limitingly, from:
- calcineurin inhibitors, particularly cyclosporin A (CsA) or FK506;
- anti-CD3 and anti- IL2R molecules and Jak3 inhibitors;
- steroids;
- agents capable of interfering with leukocyte proliferation, alkylating agents, dihydrofolate-reductase inhibitors (methotrexate);
- agents capable of interfering with cell migration.

Preferably, such active ingredients are selected from CsA, tacrolimus (FK506), FTY720, rapamycin, azathioprine, prednisone, MMF (mycophenolate mofetil), disodium mycophenolate or mycophenolic acid.

More preferably, the immunosuppressant drug will contain cyclosporin A (CsA).

It is understood that for each of said drugs, a different therapeutically effective dose will be determined on the basis of the level of efficacy, the toxicity and any other characteristic factors for each of the active ingredients. Other factors influencing the choice of therapeutically effective dose to be administered to patients are the patient's state of health, the type of disorder to which they are subject and their age etc.

In certain cases and for certain drugs, plasma levels of the immunosuppressant drug will vary from one patient to another, despite starting from an equal initial administered dose. In such cases, it will therefore be necessary to alter the dosage during treatment so as to obtain the desired plasma levels in the patient.

Hence, it is understood that for each drug, there will be a dosage interval, inside of which the therapeutically effective dose for treating the patient will be established, or within which said dose will be determined during the course of the treatment depending on the plasma levels detected. By way of example, said dose interval for cyclosporin A is typically 3-5 mg/kg of body weight twice daily, while for tacrolimus it is generally comprised of between an initial dose of 0.075 mg/kg/day and a subsequent dose allowing the maintenance of a plasma concentration of 10-20 ng/ml during the first three months of treatment and 5-10 ng/ml for the fourth month onwards. Instead, the dose interval for azathioprine is comprised of between 0.5 and 3 mg/kg/day.

The metalloporphyrins of the invention have the following general formula (I): 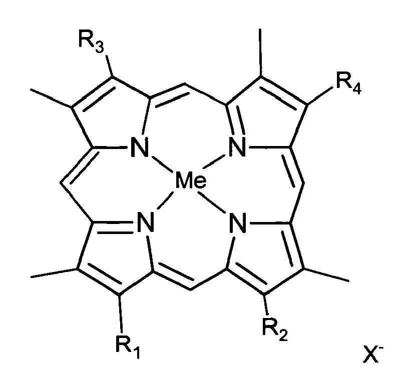 in which
Me is cobalt or zinc;
R₁ and R₂ are independently selected from -(CH₂)ₙ-COOH, - (CH₂)ₙ-COOR, - (CH₂)ₙ-CN, - (CH₂)ₙ-CONR₂, - (CH₂)ₙ-OCOR, - (CH₂)ₙ-NRCOR, - (CH₂)ₙ-COR, wherein n is 2 or 3 and the R groups are independently selected from H, linear or branched, saturated or unsaturated (C₁-C₁₀) alkyl, (C₃-C₁₀) cycloalkyl, optionally substituted (C₆-C₁₀) aryl, optionally substituted (C₁-C₃) alkyl-(C₆-C₁₀) aryl;
R₃ and R₄ are independently selected from -CH=CH₂ and -Et;
X⁻ is a counterion, such as for example chloride.

Preferably, R₁ and R₂ are both -CH₂CH₂COOH.

More preferably, R₃ and R₄ are identical.

Even more preferably, Me is cobalt (III).

Particularly preferred metalloporphyrins are cobalt protoporphyrin (CoPP) and cobalt mesoporphyrin (CoMP).

The metalloporphyrins of the invention may be prepared according to synthetic schemes known to those skilled in the art, such as those described in "Porphyrins and Metalloporphyrins", Ed. K.M. Smith, Elsevier Scientific Pub. Co. (1975) or in US patent US 5,149,697.

### BRIEF DESCRIPTION OF THE FIGURES

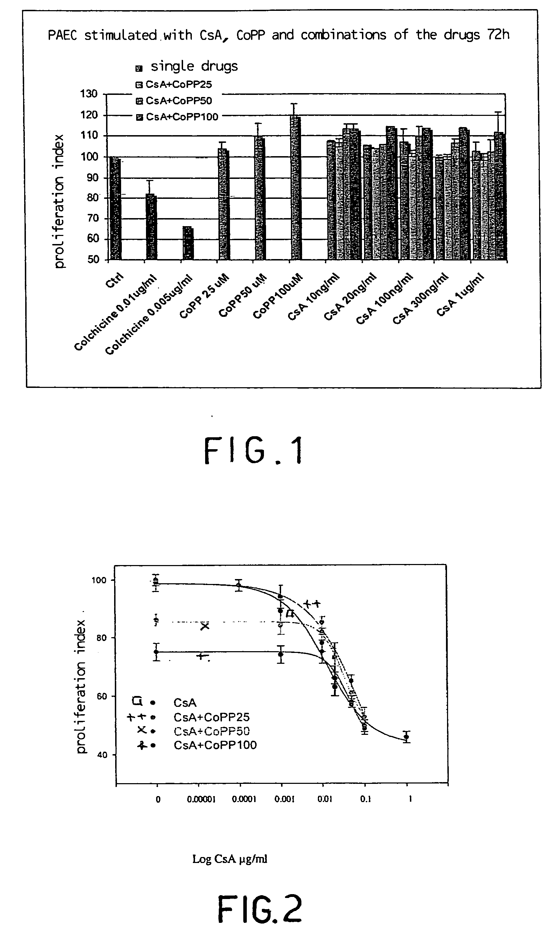

**Figure 1** shows the results of the proliferation analysis of PAEC cells treated with CoPP, cyclosporin A (CsA) and combinations thereof, by means of staining with CFSE: the proliferation obtained from the mean of two experiments is measured as the variation of the proliferation index with respect to untreated PAEC controls; PAEC cells have been treated with various concentrations of CsA, CoPP and combinations thereof.
**Figure 2** shows the results of the proliferation index analysis of PBMCs of 3 primates (W850, W915, Y214) stained with CFSE, activated for proliferation by concanavalin A and stimulated with CoPP, CsA and combinations thereof for 96 hours.

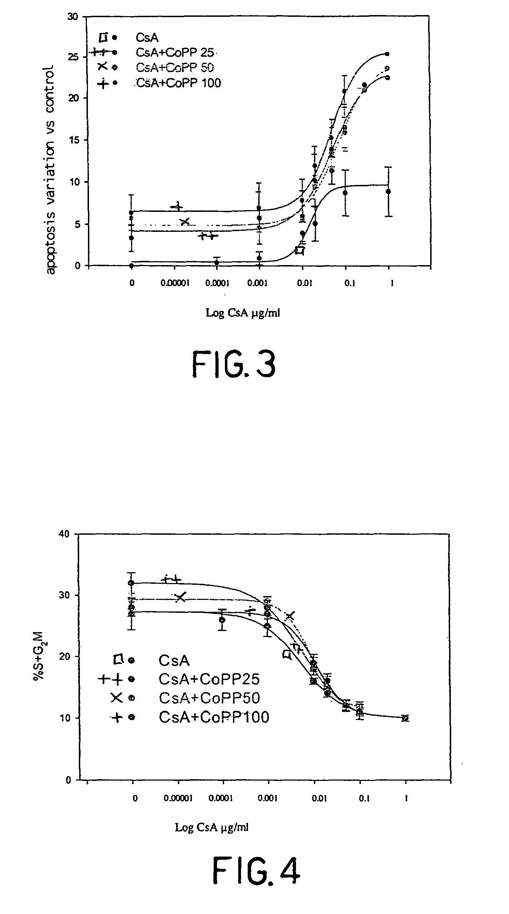

**Figure 3** shows the results of the apoptosis analysis of PBMCs of 3 primates (W850, W915, Y214) activated for proliferation by concanavalin A and treated with CsA alone or in combination with CoPP for 96 hours (n=5). Following activation, the PBMC cells have been harvested, fixed in ethanol overnight, stained with PI and analysed using a cytofluorimeter.
**Figure 4** shows the results of the analysis of the percentage of simian PBMC cells in the synthesis and cell division stage treated with CsA alone or in combination with CoPP (n=4). Following stimulation, the cells have been harvested, fixed in ethanol, stained with PI and analysed using a cytofluorimeter.

### EXPERIMENTAL SECTION

Two *in vitro* experimental models have been used. Pig aorta endothelial cells (PAEC) have been incubated with increasing doses of immunosuppressant drug with the aim of studying the effect of such drugs on the proliferation of the culture itself. For the drug under investigation, the effect of metalloporphyrins on cultured endothelial cells has been evaluated, in comparison to the harmful effects deriving from incubation of the cells with the immunosuppressant drug.

In the second model, mononucleate cells isolated from non-human primate peripheral blood (PBMC) have been used, stimulated for proliferation with concanavalin A and incubated with increasing concentrations of immunosuppressant drug alone or in combination with metalloporphyrin. In the second model it was desired to test the capacity to inhibit proliferation of lymphoid cells by the individual drug, and the combination of drug with metalloporphyrin.

### Cell proliferation analysis model by means of labelling with CFSE and cytofluorometric analysis

In order to study cell proliferation, PAECs have been labelled with CFSE and stimulated with the drug alone or in combination. CFSE is a non-fluorescent dye which diffuses passively into the cell cytoplasm. Once inside the cytoplasm, the dye is cleaved by intracellular esterases, thus becoming fluorescent. This binds strongly to cellular proteins and is retained during cellular development. At each cell division stage, CFSE is distributed evenly to daughter cells, with the result that each cellular generation will inherit half of the CFSE content. When analysed by cytofluorimetry, the sequential reduction of CFSE is visualised as distinct peaks corresponding to ever more decreasing fluorescence. PAEC proliferation has been evaluated as a proliferation index, calculated as the sum of the cells in all generations divided by the number of original parental cells (e.g. in the second generation, the parental cells would correspond to the total number of cells in the second generation divided by 2, in the third, divided by 4, because each will have given rise to 2¹, 2² cells and so on for each generation).

### HO-1 expression analysis model by means of Western Blotting

In order to evaluate the influence of the immunosuppressant drug on the expression of HO-1, following stimulation, PAEC cells have been washed in PBS and harvested in loading buffer. Equal amounts of protein of each sample have been loaded and separated by electrophoresis on a 12% polyacrylamide gel in the presence of SDS (SDS-PAGE) and then transferred onto a nitrocellulose membrane (Amersham-Pharmacia) by means of electroblotting. Heme oxygenase has been detected using a rabbit anti-heme oxygenase antibody (1:5000, Stressgen) and a sheep anti-rabbit IgG antibody horseradish peroxidase (HRP) conjugate (1:2000, Calbiochem) used as secondary antibody. The specific band has been detected by adding a chemiluminescent substrate followed by exposure to photographic film (ECL-Amersham-Pharmacia). The relative amount of HO-1 protein, normalised to the actin content of each sample, has been determined with the use of a densitometer (Pharmacia).

### Proliferation analysis model of concanavalin A activated simian PBMCs, by means of labelling with CFSE

Peripheral blood mononucleated cells (PBMC) have been isolated from samples of primate blood collected in the presence of sodium citrate, by means of sedimentation in dextran and subsequent separation on a Ficoll gradient. The cells have been labelled with 10 µM CFSE, stimulated with concanavalin A, and at the same time, CoPP, cyclosporin A, and combinations of both molecules have been added at various concentrations for 96 hours. Proliferation has been analysed using a cytofluorimeter.

### Cell cycle analysis model of simian PBMCs by means of staining with PI

Simian PBMCs, activated for proliferation by concanavalin A and stimulated with CoPP, cyclosporin A and combinations thereof for 96 hours, have been harvested and washed in PBS.

The pellet from each sample has been resuspended in 600 µl of PBS and subsequently, 1.4 ml of absolute ethanol has been added dropwise while shaking continuously. The samples have been left at -20°C overnight. Following fixation, the cells have been washed twice in PBS. The pellet has been resuspended in 500 µl of PI at a concentration of 50 µg/ml, to which 6.25 µl of Nonidet P40 and 8 µl of RNase A (1 mg/ml) have been added. The samples have been shaken vigorously and incubated for at least half an hour in the dark at room temperature. Following incubation, the samples have been analysed using a cytofluorimeter, for cell cycle analysis.

### RESULTS

### Analysis of the proliferation of PAEC cells treated with cyclosporin A alone, in combination with CoPP, or with CoPP alone, by means of CFSE labelling

To analyse the mitogenic or protective effect of CoPP we have measured the proliferation of PAECs treated by the drug by itself or in combination, by means of staining the cells with a fluorescent probe (CFSE) which identifies the various cellular generations. The results of this experiment, shown in figure 1, have indicated that CoPP increases the proliferation index in a dose dependent manner. When the drug cyclosporin A is used at concentrations lower then the therapeutic doses normally used in humans, a proliferative effect on PAEC cells is obtained, while at therapeutic concentrations in excess of 100 ng/ml, it seems to have little influence on the proliferation of the same PAEC cells. The combination with CoPP increases the proliferative capacity, which exceeds the control value even for high concentrations of cyclosporin A, but which never seems to achieve the values obtained when CoPP is used alone.

### The in vitro effects of cyclosporin A alone or in combination with CoPP in simian PBMCs

Simian PBMCs, isolated from peripheral blood, have been activated for proliferation with concanavalin A and stimulated with CoPP, CsA and combinations thereof at various concentrations for 96 hours. By means of staining with the DNA intercalator, PI, it has been possible to evaluate the apoptotic cells as a sub-diploid peak and the cells in the DNA synthesis and duplication stage (S+G2 stage) as peaks with a DNA content greater than or equal to the diploid content. The proliferation index of PBMCs has been evaluated by staining the cells with CFSE at the same time as stimulation.

### Analysis of proliferation index

Study of the proliferation of PBMCs has been performed by evaluating the variation of the proliferation index of cells treated with the drug with respect to untreated PBMC controls, to which the arbitrary value of 100 has been assigned. The proliferation index of the cells analysed by cytofluorimetry may be deduced from analysis of the distribution of the cellular populations in the various generations.

The proliferation indices of the cells treated with various concentrations of CsA alone or in association with CoPP at various concentrations are reported in figure 2, where it may be observed that a reduction in PBMC proliferation is obtained in all cases. The trend of the variation of the proliferation index as a function of increasing concentrations of CoPP is highlighted when the CsA concentration is equal to 0 (the left most point of the graph). CoPP inhibits proliferation at concentrations in excess of 25 µM. In relation to PBMCs treated with CsA, not only is an accentuation of proliferation inhibition not observed when it is in association with CoPP, but it seems that CoPP reduces the efficacy of CsA mediated inhibition.

In conclusion, it seems that the association of CsA and CoPP has no significant effect on the inhibition of concanavalin A induced PBMC proliferation.

### Apoptosis analysis

In order to study apoptosis, the percentage of cells in the sub-diploid stage has been quantified. Cyclosporin A induces apoptosis in PBMCs in a dose dependent manner, represented in figure 3 as the variation with respect to untreated PBMC controls. CsA induces apoptosis from a concentration of 10 ng/ml upwards and reaches a plateau of 9% at higher concentrations.

When PBMCs are stimulated with CsA in association with 100 µM CoPP, apoptosis is increased up to 25%, while CoPP alone induces apoptosis up to 6% at most. In conclusion, the association of CsA with CoPP potentiates the apoptosis inducing effect of the immunosuppressant drug in a synergic manner.

### Analysis of the cells in G2+S stage

In order to study the cells in G2+S stage, the percentage of cells having a DNA content greater than 2n has been quantified. As shown in figure 4, when PBMCs are treated with CsA in association with CoPP, it seems they are further forced into the G2+S stage.

### Analysis of HO-1 expression in PAECs

PAECs have been stimulated with increasing concentrations of cyclosporin A and with 50 e 100 µM CoPP for 72 hours. Following stimulation, HO-1 expression has been assessed by Western Blotting and densitometric analysis of the bands obtained. HO-1 expression is reported as the ratio of the intensity of the bands with respect to the control (table 1). The amount of protein loaded is normalised for actin. As may be observed from the table, CoPP induces HO-1 expression in a dose-dependent manner. CsA also induces HO-1 expression.

**Table 1: HO-1 expression in PAECs as the ratio of band intensity with respect to the control and normalised for actin (R)**

| | **CoPP 50** | **CoPP 100** | **CsA 0.1 ng/ml** | **CsA 1 ng/ml** | **CsA 10 ng/ml** | **CsA 100 ng/ml** | **CsA 1 µg/ml** | **CsA 10 µg/ml** |
|---|---|---|---|---|---|---|---|---|
| R | 6.0 | 4.6 | 0.9 | 1.0 | 1.1 | 1.0 | 2.6 | 4.7 |

From the above results it may be deduced that, on the one hand, the use of metalloporphyrin, through the proliferative stimulation of endothelial cells, may promote the repair of endothelial damage as a result of both damage due to ischemia/reperfusion and the initiation of rejection, and from the immunosuppressant drug itself. On the other hand, the synergic effect demonstrated by the association of the metalloporphyrin and the immunosuppressant drug in the selective induction of apoptosis on lymphocytes, allows the use of the immunosuppressant drug at a dose which is lower than the predetermined therapeutically effective dose, or the dose determined during treatment on the basis of the plasma levels of the immunosuppressant drug, for the treatment of transplant patients or patients affected by autoimmune disorders when the immunosuppressant drug is administered alone. Preferably, the dose of said immunosuppressant drug will be at least 25%, more preferably at least 30% lower than the predetermined therapeutically effective dose, or the dose determined during the course of treatment on the basis of plasma levels of immunosuppressant drug, for the treatment of transplant patients or patients affected by autoimmune disorders when the immunosuppressant drug is administered alone.

This way, it is possible to reduce the side effects resulting from use of the immunosuppressant drug, which in certain cases might lead to the suspension of treatment.

The metalloporphyrins of the invention may be used for the treatment of humans or animals.

Hence, a subject of the present invention is the use of a metalloporphyrin of formula (I) for the preparation of a remedy for a joint therapy with an immunosuppressant drug in transplant patients or patients affected by an autoimmune disorder, wherein said immunosuppressant drug is used at a dose lower then the predetermined therapeutically effective dose, or the dose determined during treatment on the basis of plasma levels of the immunosuppressant drug, for the treatment of said patients when the immunosuppressant drug is administered alone.

In consideration of the above, the use of metalloporphyrins of formula (I) for the preparation of a remedy for a joint therapy with at least one immunosuppressant drug in transplant patients or patients affected by an autoimmune disorder, wherein said immunosuppressant drug is used at a dose lower than the predetermined therapeutically effective dose, or the dose determined during treatment on the basis of plasma levels of the immunosuppressant drug, for the treatment of said patients when the immunosuppressant drug is administered alone and where said patients are constituted by a sub-population of patients having undergone treatment with an immunosuppressant drug and having demonstrated untolerated side effects, is also a subject of the present invention.

The normally used, therapeutically effective dose for any given immunosuppressant drug, is known and reported in the literature. Generally, it may vary depending on the disorder to be treated and the condition of the patient, their weight, their age etc.

The metalloporphyrin of formula (I) may be administered orally, topically or parenterally (for example, rectally or by i.v.) at a dose which may vary between 0.1 and 2 mg/kg from 1 to 4 times daily. The dosage of the metalloporphyrin of formula (I), like that of the immunosuppressant drug, will be determined on the basis of the disorder to be treated, the level of seriousness of said disorder and the patient's conditions, as defined above. Furthermore, the dose will depend on the selected route of administration. It should be considered that it might be necessary to make continual adjustments to the dosage depending on the age and weight of the patient, in addition to the seriousness of the clinical condition to be treated. The exact dose and the administration route will finally be at the discretion of the physician or veterinarian.

The metalloporphyrin and immunosuppressant drug may be used separately or combined in a pharmaceutical composition.

A further subject of the present invention is hence a kit for joint, separate or sequential administration comprising a first pharmaceutical composition of at least one immunosuppressant drug and a second pharmaceutical composition of at least one metalloporphyrin of formula (I).

A further subject of the present invention is a pharmaceutical composition comprising at least one immunosuppressant drug and at least one metalloporphyrin of formula (I) together with pharmaceutically acceptable excipients.

In such pharmaceutical compositions, the immunosuppressant drug will be as defined previously, and may be a single drug or a mixture of drugs.

Hence, the compounds to be used, according to the present invention, may be formulated for oral, buccal, parenteral, rectal or transdermal administration or in a form which is suitable for administration by inhalation or insufflation (either through the mouth or nose).

For oral administration, the pharmaceutical compositions may be, for example, in the form of tablets or capsules, prepared in the conventional manner with the aid of pharmaceutically acceptable excipients such as binding agents (for example pre-gelatinised corn starch, polyvinylpyrrolidone or hydroxypropyl methylcellulose); fillers (for example lactose, microcrystalline cellulose or calcium hydrogen phosphate); lubricants (for example magnesium stearate, talc or silica); disintegrants (for example potato starch or sodium starch glycolate); or inhibiting agents (for example sodium dodecyl sulphate). Tablets may be coated, using methods well known in the art. Liquid preparations for oral administration may be, for example, in the form of solutions, syrups or suspensions or may be as lyophilised products to be reconstituted, prior to use, with water or other suitable carriers. Such liquid preparations may be obtained using conventional methods with pharmaceutically acceptable additives such as suspension agents (for example sorbitol syrup, cellulose derivatives or hydrogenated edible fats); emulsifiers (for example lecithin or acacia); non-aqueous carriers (for example almond oil, oily esters, ethyl alcohol or fractionated vegetable oils); and preservatives (for example methyl- or propyl-p-hydroxybenzoates or sorbic acid). Preparations may also appropriately contain flavourings, colourants and sweeteners.

Preparations for oral administration may be appropriately formulated to allow the controlled release of the active ingredient.

Compositions for buccal administration may be in the form of conventionally formulated tablets or lozenges.

The compounds according to the present invention may be formulated for parenteral administration by injection. Formulations for injections may be presented in single dose form, for example in vials with added preservative. The compositions may be presented in the aforementioned form as suspensions, solutions or emulsions in oily or aqueous carriers and may contain formulary agents such as suspension agents, stabilisers and/or dispersants. Alternatively, the active ingredient may be in powder form for reconstitution, prior to use, using an appropriate carrier, for example sterile water.

According to the present invention, the compounds may also be formulated in rectal compositions such as suppositories or retention enemas, for example containing common basic suppository components such as cocoa butter or other glycerides.

In addition to the previously described compositions, the compounds may also be formulated as deposit preparations. Such long acting preparations may be administered as implants (for example subcutaneously, transcutaneously or intramuscularly) or by intramuscular injection. Thus, for example, the compounds according to the present invention, may be formulated with suitable polymeric or hydrophobic materials (for example in the form of an emulsion in a suitable oil) or ion exchange resin or as sparingly soluble derivatives, for example as a sparingly soluble salt.

## Claims

1. Use of a metalloporphyrin of formula (I): 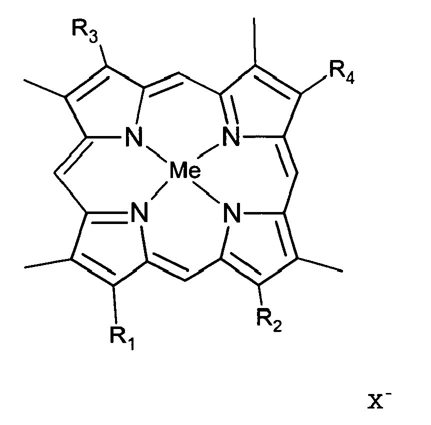 in which
Me is cobalt or zinc;
R₁ and R₂ are independently selected from - (CH₂)ₙ-COOH, - (CH₂)ₙ-COOR, - (CH₂)ₙ-CN, - (CH₂)ₙ-CONR₂, - (CH₂)ₙ-OCOR, - (CH₂)ₙ-NRCOR, - (CH₂)ₙ-COR, wherein n is 2 or 3 and the R groups are independently selected from H, linear or branched, saturated or unsaturated (C₁-C₁₀) alkyl, (C₃-C₁₀) cycloalkyl, optionally substituted (C₆-C₁₀) aryl, optionally substituted (C₁-C₃) alkyl-(C₆-C₁₀) aryl;
R₃ and R₄ are independently selected from -CH=CH₂ and -Et;
X⁻ is a counterion, for the preparation of a remedy for a joint therapy with an immunosuppressant drug in transplant patients or patients affected by an autoimmune disorder, wherein said immunosuppressant drug is used at a dose lower then the predetermined therapeutically effective dose, or the dose determined during treatment on the basis of plasma levels of the immunosuppressant drug, for the treatment of said patients when the immunosuppressant drug is administered alone.

2. The use according to claim 1, wherein R₁ and R₂ are both -CH₂CH₂COOH.

3. The use according to claims 1 or 2, wherein R₃ and R₄ are identical.

4. The use according to any of the claims 1 to 3, wherein Me is cobalt (III).

5. The use according to any of the claims 1 to 4, wherein said metalloporphyrin is cobalt protoporphyrin (CoPP) or cobalt mesoporphyrin (CoMP).

6. The use according to any of the claims 1 to 5, wherein said immunosuppressant drug is used at a dose at least 25% lower with respect to the predetermined therapeutically effective dose, or the dose determined during treatment depending on plasma levels of the immunosuppressant drug, for the treatment of said patients when the immunosuppressant drug is administered alone.

7. The use according to any of the claims 1 to 6, wherein said immunosuppressant drug is used at a dose at least 30% lower with respect to the predetermined therapeutically effective dose, or the dose determined during treatment depending on plasma levels of the immunosuppressant drug, for the treatment of said patients when the immunosuppressant drug is administered alone.

8. The use according to any of the claims 1 to 7, wherein said immunosuppressant drug comprises at least one active ingredient selected from:
• calcineurin inhibitors;
• anti-CD3 and anti- IL2R molecules and Jak3 inhibitors;
• steroids;
• agents capable of interfering with leukocyte proliferation, alkylating agents, dihydrofolate-reductase inhibitors;
• agents capable of interfering with cell migration.

9. The use according to claim 8, wherein said at least one active ingredient is selected from cyclosporin A, tacrolimus (FK506), FTY720, rapamycin, azathioprine, prednisone, MMF (mycophenolate mofetil), methotrexate, disodium mycophenolate, mycophenolic acid.

10. The use according to any of the claims 8 to 9, wherein said immunosuppressant drug contains cyclosporin A.

11. The use according to any of the claims 1 to 10, wherein said patients are constituted by a sub-population of patients having undergone treatment with an immunosuppressant drug and having demonstrated untolerated side effects.

12. A kit for joint, separate or sequential administration comprising a first pharmaceutical composition of at least one immunosuppressant drug and a second pharmaceutical composition of at least one metalloporphyrin of formula (I) as delineated in any of the claims 1 to 5.

13. The kit according to claim 12, wherein said immunosuppressant drug is as delineated in any of the claims 8 to 10.

14. A pharmaceutical composition comprising at least one immunosuppressant drug and at least one metalloporphyrin of formula (I) as delineated in any of the claims 1 to 5, together with pharmaceutically acceptable excipients.

15. The pharmaceutical composition according to claim 14, wherein said immunosuppressant drug is as delineated in any of the claims 8 to 10.

16. The pharmaceutical composition according to claims 14 or 15, said composition being adapted to oral, buccal, parenteral, rectal or transdermal administration or in a form which is suitable for administration by inhalation or insufflation (either through the mouth or nose).
